# EUROPEAN PATENT SPECIFICATION

(11) **EP 1 062 513 B1**
(45) Date of publication and mention of the grant of the patent: **07.01.2009**
(21) Application number: 99907648.2
(22) Date of filing: 15.03.1999
(51) Int. Cl.: G01N 33/569

(54) **METHOD FOR DETECTING SALMONELLA FROM AN ENRICHMENT CULTURE**
METHODE ZUR DETEKTION VON SALMONELLA AUS VORKULTUREN
PROCEDE DE DETECTION DE SALMONELLA A PARTIR D'UNE CULTURE D'ENRICHISSEMENT

(30) Priority: 13.03.1998 FI 980571
(43) Date of publication of application: 27.12.2000
(73) Proprietor: HAKALEHTO, Elias, 70110 Kuopio (FI)
(72) Inventor: HAKALEHTO, Elias, 70110 Kuopio (FI)
(74) Representative: Karvinen, Leena Maria
(86) International application number: PCT/FI1999/000192
(87) International publication number: WO 1999/047931

(56) References cited:
- WO-A1-94/28420
- GB-A- 2 234 587

## Description

### Introduction

*Salmonella* is nowadays one of the most important bacterial contaminants found in food products. Rapid adaptation capability, which is its typical feature, causes difficulties in detecting the bacterium. At present there are over 2000 characterized *Salmonella* strains of which about 100 are clinically and hygienically important. *Salmonella* is a common cause of enteric diseases for both humans and animals. The number of most important strains found during epidemics is about ten. The occurance of *Salmonella* in food often causes large amounts of people to get exposed to an infection. Finding the original source of contamination is a challenging task. Contaminated food or water provide a typical way for the contagion to spread. *Salmonella* belongs to the so called enteric bacteria. Most of the strains cause gastroenteritis.

Normally *Salmonellas* live outside the body of people or host animals in very poor growth conditions. They have to survive and retain their viability e.g. in water, where usually several other microbes compete with them for nutrients. Usually in these circumstances the cells probably develop into some form of resting cells. Once they reach good growth conditions they have to adapt quickly to the new conditions to be able to colonize e.g. the surface epithelial cells of the gastrointestinal tract.

Outside the body *Salmonella* and other enteric bacteria are usually under strong environmental stress. When the *Salmonella* or other micro-organisms get into the body with food, they soon enter the low-pH environment of the stomach, which destroys a large amount of living microbial cells. On the other hand, a low pH also dissociates appendiges on the cell surfaces, of which especially the fimbrias or corresponding structures are used for attaching onto the epithelium. Then after entering the duodenum the *Salmonellas* and other pathogenic enteric species in order to invade the body have to synthesize rapidly the building parts of the different attachment filaments and correspondingly build these appendiges on the cell surfaces. These filaments can be utilised as the basis of immunological methods for detecting microbes, because they are normally strongly immunogenic. They can also be dissociated into single molecules which are their building parts. In the same manner, we can make good use of the detection of the flagellas that many bacteria synthesize to enable them to move and the flagellin protein, the building parts of the flagellum, in the immunological detection of microbes.

Normally, before using an immunological detection method, the *Salmonella* culture or sample or the culture of enteric bacteria, other bacteria or other micro-organisms must be selectively grown and the desired microbes enriched.

Normally in an enrichment cultivation the sample or culture is usually grown long enough for the number of cells to manifoldly outgrow that of the original sample, usually for at least 12 hours. It is generally supposed that also the amount of antigens increase in about direct ratio to the amount of cells or cell mass.

One of the key goals of food, water and other environmental hygiene is to prevent *Salmonella* bacterial from spreading. For this reason, the detection of *Salmonella* and other similar microbes is an important and broadening field of research and economic activity. The problem in using the traditional bacterial cultivation methods for diagnostics of the *Salmonella* bacteria and other pathogens is the long time needed to attain the results. This causes great expenses e.g. in food industry, where the products often need to be stored up while waiting for the results of the hygiene control or withdrawn from the market or distribution, if the results show contamination with *Salmonella* or other bacteria. Research and development work directed to microbial determinations has recently concentrated on finding more rapid detection methods for microbes.

For clinical sampling in hospitals and for the hygiene mapping of antibiotic resistant microbial strains, more rapid, more reliable and more effective methods are required. These methods should at the same time be useful also for detecting microbes in as simple conditions as possible, even outside laboratories.

Food industry also needs new rapid detection methods and fast methods of enrichment to maintain product safety, shorten storage time and control raw materials. Likewise, different water and environmental analysis, the significance of which has recently grown, need these methods urgently.

### Background of the Invention

When detecting microbes from e.g. clinical samples, food samples or environmental samples the microbial concentrations in the original sample are usually so low that so called enrichment methods are needed. These methods increase the amount and concentration of the detected mibrobes in the sample. Microbe specific cultivation methods, which usually involve the use of a selective factor to prevent the multiplication of other microbes, are used. This factor can be a chemical substance, an antibiotic or an equivalent or some physical factor such as the partial pressure of a gas. The pH can also be a selective factor. Often the synergy of the different selective factors can be used in the enrichment culture of the desired microbe in a selection.

The need of having to use enrichment methods in detecting microbes means a loss of time and therefore the shortening of the time used for these procedures is most desirable.

The specific microbial identification often uses antibodies that are produced in animals or cell cultures (immunological methods). They are often used to detect microbes from enrichment cultures, for example. In such a case, the problem may be that the user of the test does not know for sure, if the culture in concern or other sample and the cells in it have enough antigenic molecules for the detection.

The invention can be applied in large scale for monitoring *Salmonella* in foodstuffs. For example in hygiene controls in the meat industry *Salmonella* often exists in so low percentages that the direct detecting using the immunological analysis is with present methods impossible. In these cases an enrichment culture in liquid medium for at least 24 hours is often needed. The enrichment process often divides into two phases: the pre-enrichment stage and the actual enrichment stage. By controlling the conditions of the pre-enrichment growth of the possible *Salmonella* bacteria and the expression of its desired antigens in the sample can be speeded up. Adjusting the conditions of the cultivation also helps to exclude the possibility of cross-reacting, disturbing strains.The enrichment culture is used in the same way as a help to detect and identify many other bacteria and also micro-organisms.

Because the invention makes the process of detecting *Salmonella* faster, it can well serve e.g. meat industry and clinical diagnostics, which are in need of rapid methods. In industry the delivery of the products is often put into practice before the microbiological monitoring results are complete. This can lead into major losses if contamination should occur. The rapid methods would make possible to remove the spoiled products early enough.

A previous publication (GB 2 234 587A) describes a method using lipopolysaccharide (LPS) molecules as target antigens. In said method the fastest detection of *E. coli* takes place in 5 + 2 hours. The method is directly dependent on achieving high enough bacterial concentrations for detection purposes, because the amount of the LPS is proportional to the cell number.

Previous methods for the detection of *Salmonella* and other enteric bacteria using immunological methods are described in WO 92/06197, WO 94/28163 and WO 94/28420. They rely on the population cell growth as the determinative factor behind improved detection results. In WO 92/06197 the bacterial detection is based on the epitopes of the fimbrial sites. The method is, however, exploiting cultivation times such as 24 hours or overnight.

### Description of the Invention.

When growing bacteria species of the genus *Salmonella* and other enteric bacteria on selective nutrient media we noticed that they produced lots of specific antigenic molecules already before the actual microbial population growth, based on the number of cells, was at its maximum. In fact, the concentration of antigen that the specific antibody could detect on the cell surfaces had substantially lowered from its record figures as the cell growth in the enrichment culture grew near its maximum. For this reason the use of an immunological detection method can take place earlier than in the presently known methods, directly following the so called Lag phase before the number of cells, defined with e.g. the calculation methods based on the colony count, has markedly increased. For example, the temperature, the composition of the nutrient medium, antibodies or other selective molecules and the control of partial pressures of various gases can be used as selective factors in the enrichment culture.

According to the method of this invention, the identification process of microbes from various samples can be made more rapid by making use of the changes in the expressions of the different surface structures of the microbes according to the changes in the growth phase and growth conditions. By exploiting the "enhanced" enrichment of antigens we could e.g. observe the increased concentrations of type 1 fimbrial antigens in 3 - 10 hours from the onset of the cultivation (Examples 4 - 5). These fimbrias or their building parts, which the microbes produce e.g, to enable them to attach to the epithelial cells of the alimentary tract, can be made use of in methods for detecting microbes more rapidly than in the methods according to the present knowledge.

In one possible form of application, after a suitable period of enrichment it is possible to detect the microbes by taking advantage of filter extraction (Finnish patent No. 93742). The immunological detection itself can be carried out with an immunostrip, the ELISA-method, a luminometric method or other corresponding apparatus or method using antibodies, which are suitable specifically for extracted surface structures. This procedure may, in theory, add the sensitivity of the detection when the amount of cells is still relatively small.

In theory, the attachment characteristics could be made use of also in coating the plunger of the injection syringe used in taking the sample (US. Patent No. 5,846,209). The plunger used in taking the sample could then be changed into a plunger coated with specific antibodies against to the attachment molecules or molecules that imitate the object of the attachment molecules of the searched bacteria. When handling fluid samples the plunger need not be changed. Once the microbes are brought into suitable growth conditions they begin to express their attachment molecules and attach with them to the molecules on the surface of the plunger. The attachment is verified with some suitable method (e.g. electrically or optically).

Examples 4 - 5 describe the reactions of a peptide antibody, produced against different *Salmonella* strains, with bacterial cultures in different growth phases. On the basis of the results from these experiments we were able to demonstrate high immunological reaction levels already before the beginning of the actual logarithmic growth phase. When producing antibody to the fimbrial proteins and to peptides derived from them, we noticed strong immunological reactivities already in 3 - 5 hours with antibodies produced against fimbrial peptides (Examples 4 - 5). The results implicate that the *Salmonella* type 1 fimbrial sequence, used in the experiment as source material in the production of synthetic peptides, would express outstandingly strongly already before the actual logarithmic growth phase or immediately at its beginning.

When examining the growth of the bacterial cultures on different media and in different growth conditions, we noticed that e.g. the growth of a *S*. *enteritidis*-culture in a selective medium (RVS) was at its strongest after 3 - 6 hours the temperature being +37°C and the mass of the cultivation grew close to its maximum in 8 hours (Example 4).

The basis of the phenomenon described above is the theory that reaching favourable conditions in the intestines of man or a warm-blooded animal the food-borne *Salmonella* bacterium or other enteric bacterium firstly produces fimbrias and other molecular structures required for attachment, in order to be able to stick to this favourable environment, where nutrients are readily available. It is also possible that already in the stationary phase cells the attachment proteins or their precursors are at least partially prepared in the cytoplasm or in a corresponding site from where they can be mobilized in the fastest way from the point of view of the bacterial cell.

### Example 1: Cultivation of Salmonella on RVS medium

*Salmonella enteriditis* strain 9,12:-g, m:-, phage type 4 (IHS 59813) and *Salmonella typhimurium* strain 4,5,12:i:1,2, phage type 1 (IHS 59929) were kept at 37 °C in THG medium (5% tryptone, 2.5% yeast extract and 1% glucose) and seeded in the intervals of two weeks during the experiment. The *Salmonella* strains were obtained from the National Public Health Institute (Helsinki, Finland). Cultivation was started from 3 - 4 days old culture by inoculating 5% of the culture to a fresh RVS medium (Rappaport-vassiliadis soya peptone broth, Oxoid, England), which is selective for *Salmonella*. The RVS suspension was cultivated in shaked Erlenmeyer flasks (each 100 ml) at two different temperatures: +37 °C and +43 °C. Samples were taken every hour.

### Example 2: Description of the peptide

The sequence in the peptide synthesis was traced from the *Salmonella typhimurium* type 1 fimbriae. In order to select a specific sequence differing from the corresponding *E*.*coli* type 1 fimbriae, the two sequences were compared with each other. The sequence of 18 amino acids Ala Ser Phe Thr Ala Ile Gly Asp Thr Thr Ala Gln Val Pro Phe Ser Ile Val was selected. The peptide was synthetized as molecules, in which 4 identical peptides were joined together from one end forming thus a multiple-antigen peptide (MAP). The peptides were synthetized with Millipore PerSeptive 9050 Plus automated peptide synthetizer and with Fmoc synthesis strategy. Fmoc-Lys(Fmoc)-OH was used as the backbone for the branched structure.

### Example 3: Immunizations

Peptides were used for immunization without any conjugation to carrier molecules. Rabbits were immunized with 500 µg of MAP-peptide injected subcataneously. The immunization solution contained also Freund's complete adjuvant. The boosters were injected in intervals of two weeks. The solution contained also Freund's incomplete adjuvant. Together with the immunizations the rabbits were bleeded in order to take serum samples, which were stored in the freezer (-20 °C).

### Example 4:

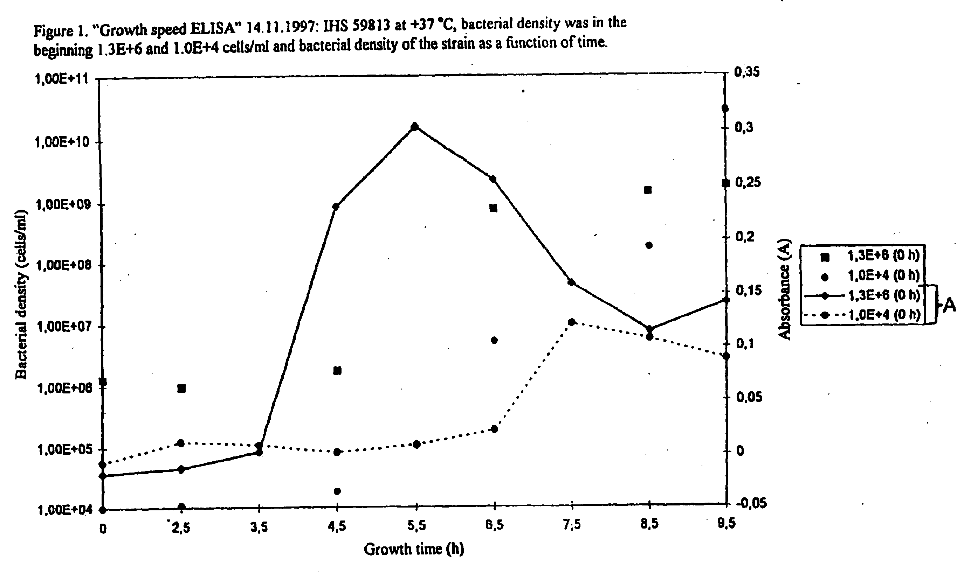

For studying the reactions of the anti-peptide antibody against bacterial cultures in different growth phases, a "growth speed-ELISA" experiment was carried out using two bacterial cultures differing from each other in cell density. *Salmonella enteriditis* strain IHS 59813 was used in the experiment, the growth medium was RVS broth and the temperature +37 °C. Plate cultures were started simultaneously with ELISA meassurements. The bacterial densities were in the beginning of the experiment 1.3 E+6 and 1.0 E+4. Results from the ELISA experiment and the plate counts are presented in Table 1 and in Figure 1. "Growth speed ELISA" was carried out as follows:
1. The microtiter plates were treated with glutaraldehyde:
   a) 150 µg of 0,5% glutaraldehyde was added to the wells and
   b) the microplate was incubated at room temperature in a shaker_for 15 minutes.
2. Washes: The glutaraldehyde was poured from the wells and the wells were washed twice with 200 µl of 1 x ELISA washing solution (5mM Tris + 0.15 M NaCl + 0.05 Tween20).
3. The antigen was added to the desired wells as an Assay Buffer dilution (1:1) 50 µl per well (Assay Buffer PBS + 1%BSA + 0.05%NaCL + 1mM EDTA).
   Into the zero wells, where no antigen was added, 50 µl 1 x washing solution was pipetted per well. Thenafter, the microplate was wrapped in a folio and incubated overnight in a cold room.
4. On the following day_the wells were poured empty and washed three times as described above.
5. For blocking 1.5% BSA/TBS solution was added to the wells (200µl/well) and the microplate was incubated on a shaker for one hour.
6. The wells were washed as in 4.
7. The primary antibodies were added (peptide serum H463):
   a) Serum dilution (1:100) was pipetted (50 µl per well).
   b) The plate was incubated on a shaker for 0.5 hours at room temperature.
8. The wells were washed four times as above.
9. The secondary antibodies were added:
   a) Secondary antibody (anti-rabbit) dilution 1:1000 was pipetted 50 µl per well.
   b) The plate was incubated on a shaker for 0.5 hours at room temperature.
10. The plates were washed three times as above and once with Afos Buffer (200 µl/well).
11. Colour solution (pNPP) was pipetted (50 µl/well) to the plate.
12. Absorbance was measured with an ELISA-reader (wave length 405 nm) after 15 minutes, half an hour, one and a half hours and three hours after the addition of colour.

**Table 1. The absorbance figures for IHS 59813 antigen (one hour after the addition of colour) and bacterial densities as the function of time.**

| Bacterial density in the beginning 1.3*10⁶ | | | | | | | | | |
|---|---|---|---|---|---|---|---|---|---|
| Time | 0 h | 2.5 h | 3.5 h | 4.5 h | 5.5 h | 6.5 h | 7.5 h | 8.5 h | 9.5 h |
| 0-absorbance | 0.245 | 0.245 | 0.245 | 0.232 | 0.227 | 0.227 | 0.227 | 0.230 | 0.234 |
| Absorbance | 0.228 | 0.233 | 0.248 | 0.464 | 0.531 | 0.483 | 0.387 | 0.345 | 0.376 |
| Bacterial density | 1.3*10⁶ | 9.7*10⁵ | | 1.8*10⁶ | | 7.7*10⁸ | | 1.3*10⁹ | 1.8*10⁹ |

| Bacterial density in the beginning 1.0*10⁴ | | | | | | | | | |
|---|---|---|---|---|---|---|---|---|---|
| Time | 0 h | 2.5 h | 3.5 h | 4.5 h | 5.5 h | 6.5 h | 7.5 h | 8.5 h | 9.5 h |
| 0-absorbance | 0.222 | 0.226 | 0.227 | O.227 | 0.227 | O.227 | 0.225 | 0.225 | 0.225 |
| Absorbance | 0.216 | 0.238 | 0.235 | 0.229 | 0.218 | 0.249 | 0.346 | 0.332 | 0.314 |
| Bacterial density | 1.0*10⁴ | 1.1*10⁴ | | 1.9*10⁴ | | 5.3*10⁶ | | 1.8*10⁸ | 2.7*10¹⁰ |

### Example 5:

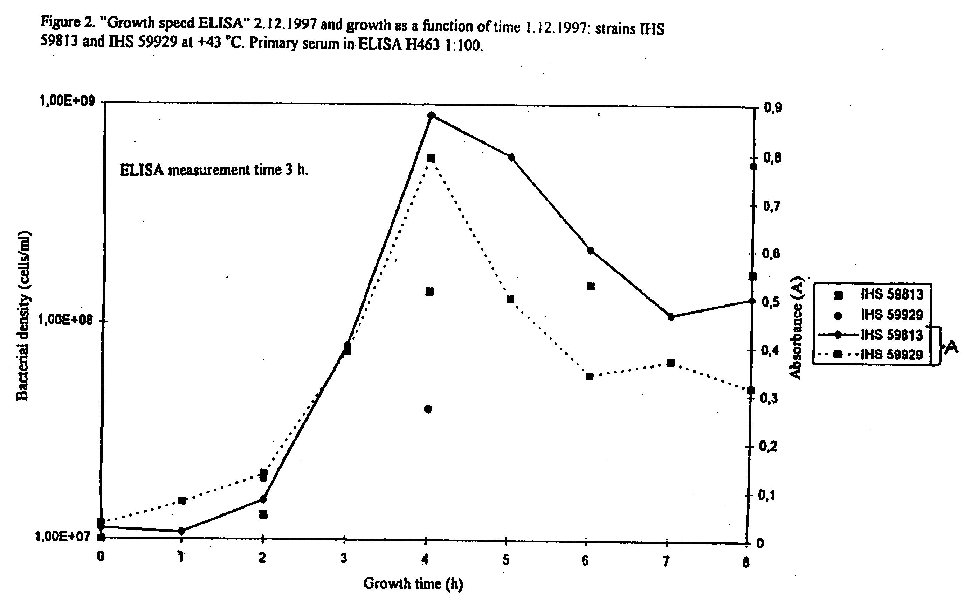

For studying the reactions of the anti-peptide antibody against bacterial cultures in different growth phases, a "growth speed-ELISA" experiment was carried out using two different *Salmonella* strains (IHS 59813 ans IHS 59929). The growth medium was RVS broth and the cultivation temperature 43 °C. Plate cultures were started simultaneously with the ELISA experiment. The experiment was carried out as described in the Example 4. The results are presented in the Table 2 and Figure 2.

**Table 2. The absorbance figures for IHS 59813 antigen and IHS 59929 antigen (three hours after the addition of colour) and bacterial densities as the function of time.**

| *Salmonella* strain IHS 59813 | | | | | | | | | |
|---|---|---|---|---|---|---|---|---|---|
| Time | 0 h | 1 h | 2 h | 3 h | 4 h | 5 h | 6 h | 7 h | 8 h |
| 0-absorbance | 0.346 | 0.346 | 0.346 | 0.336 | 0.323 | 0.323 | 0.323 | 0.357 | 0.355 |
| Absorbance | 0.369 | 0.362 | 0.428 | 0.739 | 1.202 | 1.120 | 0.928 | 0.826 | 0.859 |
| Bacterial density | 1.0*10⁷ | | 1.3*10⁸ | | 1.4*10⁸ | | 1.5*10⁸ | | 1.7*10⁸ |

| *Salmonella* strain IHS 59929 | | | | | | | | | |
|---|---|---|---|---|---|---|---|---|---|
| Time | 0 h | 1 h | 2 h | 3 h | 4 h | 5 h | 6 h | 7 h | 8 h |
| 0-absorbance | 0.360 | 0.350 | 0.344 | 0.344 | 0.344 | 0.367 | 0.355 | 0.355 | 0.355 |
| Absorbance | 0.392 | 0.427 | 0.480 | 0.733 | 1.136 | 0.867 | 0.697 | 0.726 | 0.671 |
| Bacterial density | 7.7*10⁶ | | 1.9*10⁷ | | 4.0*10⁷ | | 2.2*10⁸ | | 5.5*10⁸ |

### Literature

Hakalehto, E. Finnish patend n:o 93742, Menetelmä ja laite solujen osoittamiseksi, (A method and an apparatus for detecting cells), 1995.
Hakalehto, E. US. Patent n:o 5,846,209, Syringe comprising an adhering substrate for microbes, 1998.

## Claims

1. A microbiological determination method, **characterized in, that** that belong to the genus Salmonella bacteria are detected from their cultivation medium clearly prior to the peak of the population growth using the proteinaceous fimbrial antigens which the cells express after their inoculation to the medium, before the logarithmic growth phase or in the beginning of it.

2. A method according to the claim 1, **characterized in, that** the microbial antigens are detected immunologically using antibodies directly after the growth of the culture by cell division has been started.

3. A method according to any of the claims 1-2, **characterized in, that** the detected bacteria are enteric bacteria.

4. A method according to any of the claims 1-3 **characterized in, that** the detected antigens are type 1 fimbrial proteins.

5. A method according to any of the claims 1-4, **characterized in, that** the microbial antigens are detected with antibodies, which have been produced against the synthetic peptide Ala Ser Phe Thr Ala Ile Gly Asp Thr Thr Ala Gln Val Pro Phe Ser Ile Val or a derivative thereof

6. A method according to one or several of the claims 1-5, **characterized in that** the bacteria are cultivated prior to the detection 3-10 hours.

7. A method according to any of the claims 1-6, **characterized in, that** the microbes are incubated prior to the immunological detection in their optimal growth temperature.

8. A method according to the claim 7, **characterized in, that** the microbes are incubated prior to the detection at temperatures about 37 °C.

## Patentansprüche

1. Mikrobiologisches Bestimmungsverfahren, **dadurch gekennzeichnet, dass** die Bakterien der Gattung *Salmonella* deutlich vor dem Höhepunkt des Populationswachstums in ihrem Kultivierungsnährboden ermittelt werden, und zwar mit Hilfe der proteinösen fimbrialen Antigene, die die Zellen nach ihrer Inokulation an das Medium geben, vor der logarithmischen Wachstumsphase oder zu deren Beginn.

2. Verfahren nach Anspruch 1, **dadurch gekennzeichnet, dass** die mikrobischen Antigene immunologisch ermittelt werden, indem Antikörper direkt nach dem Wachstum der Kultur verwendet werden, wenn die Zellteilung begonnen hat.

3. Verfahren nach einem der Ansprüche 1-2, **dadurch gekennzeichnet, dass** die ermittelten Bakterien enterale Bakterien sind

4. Verfahren nach einem der Ansprüche 1-3, **dadurch gekennzeichnet, dass** die ermittelten Antigene zum Typ 1 der fimbrialen Proteine gehören.

5. Verfahren nach einem der Ansprüche 1-4, **dadurch gekennzeichnet, dass** die mikrobischen Antigene mit Antikörpern ermittelt werden, die gegen das synthetische Peptid Ala Ser Phe Thr Ala Ile Gly Asp Thr Thr Ala Gln Val Pro Phe Ser Ile Val oder gegen dessen Derivat gebildet worden sind.

6. Verfahren nach einem oder mehreren der Ansprüche 1-5, **dadurch gekennzeichnet, dass** die Bakterien 3 -10 Stunden vor der Ermittlung kultiviert werden.

7. Verfahren nach einem der Ansprüche 1-6, **dadurch gekennzeichnet, dass** die Mikroben vor der immunologischen Ermittlung in ihrer optimalen Wachstumstemperatur inkubiert werden.

8. Verfahren nach Anspruch 7, **dadurch gekennzeichnet, dass** die Mikroben vor der Ermittlung bei Temperaturen von etwa 37 °C inkubiert werden.

## Revendications

1. Procédé de détermination microbiologique, **caractérisé en ce que** les bactéries du genre *Salmonella* sont détectées à partir de leur substance de culture bien avant le pic de croissance de la population en utilisant les antigènes fimbriaux protéiques dont les cellules s'expriment après leur inoculation à la substance, avant la phase de croissance logarithmique ou au début de celle-ci.

2. Procédé selon la revendication 1, **caractérisé en ce que** les antigènes microbiens sont détectés de façon immunologique en utilisant des anticorps directement après que la croissance de la culture par division cellulaire a été initiée.

3. Procédé selon l'une quelconque des revendications 1-2, **caractérisé en ce que** les bactéries détectées sont des bactéries entériques.

4. Procédé selon l'une quelconque des revendications 1-3, **caractérisé en ce que** les antigènes détectés sont des protéines fimbriales de type 1.

5. Procédé selon l'une quelconque des revendications 1-4, **caractérisé en ce que** les antigènes microbiens sont détectés avec des anticorps, lesquels ont été produits relativement au peptide synthétique Ala Ser Phe Thr Ala Ile Gly Asp Thr Thr Ala Gln Val Pro Phe Ser Ile Val ou un dérivé de ce peptide.

6. Procédé selon l'une quelconque ou plusieurs des revendications 1-5, **caractérisé en ce que** les bactéries sont cultivées 3 à 10 heures avant la détection.

7. Procédé selon l'une quelconque des revendications 1-6, **caractérisé en ce que** les microbes sont incubés avant la détection immunologique dans leur température optimale de croissance.

8. Procédé selon la revendication 7, **caractérisé en ce que** les microbes sont incubés avant la détection à des températures d'environ 37 °C.
